# EUROPEAN PATENT APPLICATION

(11) **EP 3 106 077 A1**
(43) Date of publication of application: **21.12.2016**
(21) Application number: 15749039.2
(22) Date of filing: 29.01.2015
(51) Int. Cl.: A61B 1/00, G02B 23/24

(54) **MANIPULATOR AND MANIPULATOR SYSTEM**

(30) Priority: 13.02.2014 JP 2014025268
(71) Applicant: Olympus Corporation, Tokyo 192-8507 (JP)
(72) Inventor: YOSHIMURA, Katsuhiko, Tokyo 151-0072 (JP)
(74) Representative: Gunzelmann, Rainer
(86) International application number: PCT/JP2015/052479
(87) International publication number: WO 2015/122283

(57) **Abstract**

An object is to provide an endoscope with which the operator is less uncomfortable.

The manipulator 1 is characterized by including an insert part 2 capable of being inserted into the body cavity, a turning part 3 for turning the insert part 2, an operating unit 5 for operating the insert part 2 and turning part 3, a driving unit 4 for driving the insert part 2 and turning part 3, and a control unit 6 for controlling the driving unit 4in association with operation of the operating unit 3, wherein the insert part 2 includes a bendable distal-end action part 23, the turning part 3 includes a turning action part 34 for turning the insert part 2 and a passive part 32 that is placed into passive action in association with the turning action of the turning action part 34, the driving unit 6 includes a distal-end driving unit 41 for generating a driving force adapted to bend the distal-end action part 23 and a turning driving unit 42 for generating a driving force adapted to turn the turning action part, and the control unit 6 controls the distal-end driving unit 41 and turning driving unit 42 in association with operation of the operating unit 41.

## Description

### Technical Field

The present invention relates to a manipulator and a manipulator system inserted through the body cavity.

### Background Art

There has been a manipulator widely used in which an elongate insert part is inserted through the body cavity to haul and bend the curving part of the distal end of the insert part as by wires for the purpose of viewing, or applying treatments to, organs within the body cavity.

For instance, Patent Literature 1 discloses the technology of locating an advanceable/retractable member advanceably and retractably in the curving part of an insert part by way of a guide. The advanceable/retractable member is moved and adjusted in such a way as to be advanceable/retractable through the curving part of the insert part for the purpose of positioning the curving part of the insert part selectively or in a substantially linear state or, alternatively, setting it in such a way as to be curved freely.

Patent Literature 2, on the other hand, discloses the technology of achieving two-stage curving within the interior of the body for a wider viewable range.

### Citation List

### Patent Literature

Patent Literature 1: JP(A) 2005-74148
Patent Literature 2: JP(A) 2010-252842

### Summary of Invention

### Technical Problem

Referring to the prior art manipulator disclosed in Patent Publication 1, while an operator views or treats a subject of interest, it is often required for the operator to view or treat that subject of interest from another angle. When the operator makes the manipulator curve in situ, the manipulator may be likely to curve just in front of the subject of interest with the result that the subject of interest comes out of sight. To let the subject of interest come in sight, therefore, it is required to move the manipulator by a large amount outside the body. As a result, the manipulator to be moved often interferes with other manipulator, or the manipulator is moved by a large amount with the result that the operator often grows fatigued.

With the conventional manipulator set forth in Patent Publication 2, its movement outside the body may be kept small, but more wires must be used in the insert part so as to be well compatible with complicated movements in the interior of the body. To this end, many more wires must be stored in the insert part to be inserted through the body, resulting in an increase in the diameter of the insert part and being more invasive of the patient.

Having been made with the situations in mind, the present invention has for its object to provide a manipulator and a manipulator system that can take approaches to a subject of interest from varying angles in simple operation.

### Solution to Problem

The manipulator according to one embodiment includes
an insert part capable of being inserted into the body cavity,
a turning part for turning the insert part,
an operating unit for operating the insert part and the turning part,
a driving unit for driving the insert part and the turning part,
and
a control unit for controlling the driving unit in association with operation of the operating unit,
wherein
the insert part includes a bendable distal-end action part,
the turning part includes a turning action part for turning the insert part, and a passive part that is placed into passive action in association with turning action of the turning action part,
the driving unit includes a distal-end driving unit for generating a driving force adapted to bend the distal-end action part and a turning driving unit for generating a driving force adapted to turn the turning action part, and
the control unit controls the distal-end driving unit and the turning driving unit in association with operation of the operating unit.

The manipulator according to one embodiment includes a extension/contraction part for extending and contracting the insert part,
wherein the driving unit includes an extension/contraction driving unit for generating a driving force adapted to extend and contract the extension/contraction part,
and
the control unit controls the extension/contraction driving unit in association with operation of the operating unit.

In the manipulator according to one embodiment, the turning action part, the passive part, the extension/contraction part and the distal-end action part are located in this order from the operating unit side.

In the manipulator according to one embodiment, the operating unit includes a distance adjustment unit for adjusting a distance between a distal end of the insert part and a subject of interest, and the control unit controls the driving unit in association with a distance set by the distance adjustment unit.

The manipulator according to one embodiment includes a distance measurement unit for sensing a distance between a distal end of the insert part and a subject of interest, and wherein the control unit controls the driving unit in association with information sensed by the distance measurement unit.

In the manipulator according to one embodiment, the insert part is provided at a distal end on the turning part side with a stopper having a diameter larger than that of the insert part.

In the manipulator according to one embodiment, the operating unit includes a mode switchover instruction unit adapted to switch between a manual mode and a control mode in which the control unit controls the driving unit in association with operation of the operating unit.

In the manipulator according to one embodiment, the control unit implements control such that the distal-end part turns to the subject of interest after control.

The manipulator system according to one embodiment includes the manipulator including an endoscope, an image processor adapted to apply image processing to an image signal obtained from the endoscope, and a monitor adapted to display an image signal transmitted from the image processor.

### Advantageous Effects of Invention

With the manipulator and manipulator system according to one embodiment, it is possible to take approaches to a subject of interest from varying angles in simple operation.

### Brief Description of Drawings

Fig. 1 is a schematic view of the manipulator 1 according to the first embodiment.
Fig. 2 is a schematic enlarged view of the insert part 2 according to the first embodiment.
Fig. 3 is a schematic enlarged view of the turning part 3 according to the first embodiment.
Fig. 4 is a schematic enlarged view of the driving unit 4 and operating unit 5 according to the first embodiment.
Fig. 5 is a block diagram for the manipulator 1 according to the first embodiment.
Fig. 6 is a control flowchart for the manipulator 1 according to the first embodiment.
Fig. 7 is illustrative in schematic of a state of the manipulator according to the first embodiment before electrically operated mode control.
Fig. 8 is illustrative in schematic of a state of the manipulator 1 according to the first embodiment just after electrically operated mode control.
Fig. 9 is illustrative in schematic of a state of the manipulator 1 according to the first embodiment upon a transition from after electrically operated mode control to a manually operated mode.
Fig. 10 is a schematic view of the manipulator 1 according to the second embodiment.
Fig. 11 is a schematic enlarged view of the extension/contraction part 7 according to the second embodiment.
Fig. 12 is a schematic view of one example of the operation of the extension/contraction part 7 according to the second embodiment.
Fig. 13 is a schematic view of another example of the extension/contraction part 7.
Fig. 14 is a block diagram for the manipulator 1 according to the second embodiment.
Fig. 15 is a control flowchart for the manipulator 1 according to the second embodiment.
Fig. 16 is a schematic view of a state of the manipulator 1 according to the second embodiment before electrically operated mode control.
Fig. 17 is a schematic view of a state of the manipulator 1 according to the second embodiment after electrically operated mode control of the turning driving unit.
Fig. 18 is a schematic view of a state of the manipulator 1 according to the second embodiment after electrically operated mode control of the extension/contraction driving unit.
Fig. 19 is a schematic view of the geometrical configuration for electrically operated mode control of the manipulator 1 according to the second embodiment.
Fig. 20 is a schematic view of the manipulator 1 according to the third embodiment.
Fig. 21 is a block diagram for the manipulator 1 according to the third embodiment.
Fig. 22 is a control flowchart for the manipulator 1 according to the third embodiment.
Fig. 23 is a schematic view of another example of the manipulator 1.
Fig. 24 is a schematic view of yet another example of the manipulator 1.
Fig. 25 is a schematic view of the manipulator system 10 according to one embodiment.

### Description of Embodiments

The manipulator 1 according to one embodiment and how to control the manipulator will now be explained.

The manipulator 1 according to the embodiment described herein is used for laparoscopic surgery or the like wherein medical instruments are inserted into the abdomen or the like of a patient through multiple incisions cut open to view and treat an affected site while checking up on taken images.

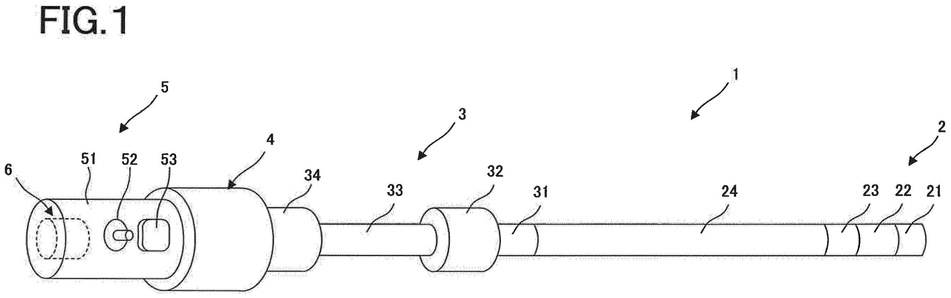

Fig. 1 is a schematic view of the manipulator 1 according to the first embodiment.

The manipulator 1 according to the first embodiment includes, in order from its distal end to its proximal end, an insert part 2, a turning part 3, a driving unit 4, and an operating unit 5. A control unit 6 is preferably stored in the driving unit 4 or the operating unit 5.

The insert part 2 includes, in order from its distal end side, a distal-end part 21, a first insert part 22, a distal-end action part 23, and a second insert part 24, and the turning part 3 includes, in order from its distal end side, a first turning part 31, a passive part 32, a second turning part 33, and a turning action part 34. The operating unit 5 includes a grip 51, an operation instruction unit 52, and a mode switchover instruction unit 53.

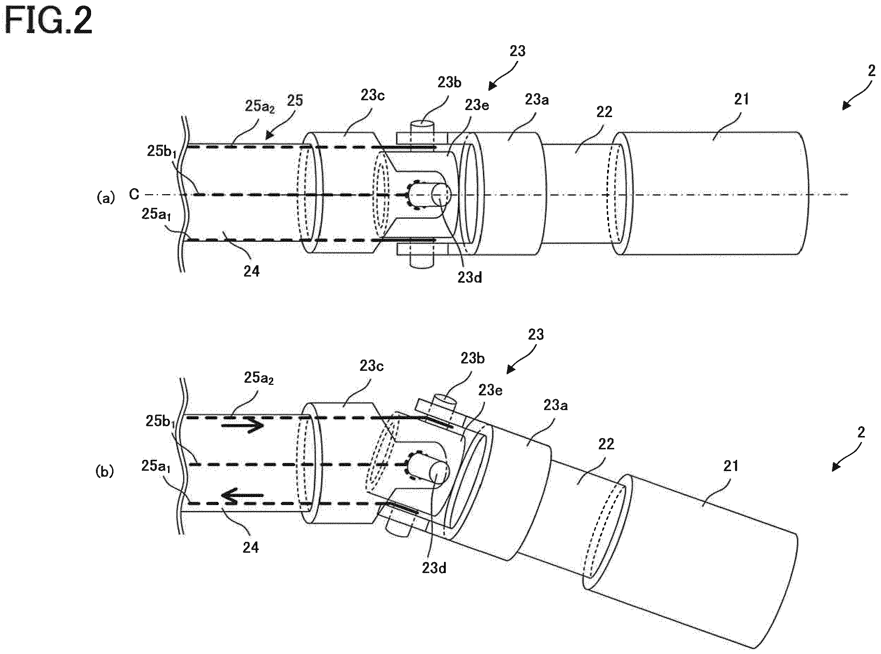

Fig. 2 is a schematic enlarged view of the insert part 2 in the manipulator 1 according to the first embodiment: Fig. 2(a) is a schematic enlarged view of the insert part 2 in a normal or linear state, and Fig. 2(b) is a schematic enlarged view of the insert part 2 in a bending state.

The distal-end part 21 is provided at the distal end of the manipulator 1, and has medical instruments incorporated inside. Taking an endoscope as an example, the distal-end part 21 may be provided inside with an imaging device for taking images of a subject of interest, a lighting device for lighting the subject of interest, etc., and taking a treatment tool as an example, it may be provided inside with forceps and a scalpel for treating the subject of interest, etc. Note here that both the endoscope and the treatment tool may be built in the distal-end part 21.

The distal-end action part 23 is made up of a universal joint or the like. In the embodiment described herein, the distal-end action part 23 has a structure in which a first distal-end shaft member 23b adapted to rotatably support a tubular first distal-end trunk part 23a coupled to the first insert part 22 and a second distal-end shaft member 23d adapted to rotatably support a second distal-end trunk part 23c coupled to the second insert part 24 are attached to a tubular distal-end articulating piece 23e in the shape of a cross.

The first distal-end shaft member 23b is provided on one side with a first distal-end wire 25a₁ and on the other side with a distal-end wire 25a₂ with the distal-end articulating piece 23e in between. Likewise, the second distal-end shaft member 23d is provided on one side with a third distal-end wire 25b₁ and on the other side with a fourth distal-end wire 25b₂ (not shown) with the distal-end articulating piece 23e in between.

The first distal-end wires 25a₁ and the second distal-end wire 25a₂ are connected together such that they can be pulled or let out by the driver 4. As the first distal-end wire 25a₁ is pulled toward the driver 4 side, it causes the second distal-end wire 25a₂ to be let out of the driver 4 side, and as the second distal-end wire 25a₂ is pulled toward the driver 4 side, it causes the first distal-end wire 25a₁ to be let out of the driver 4 side. Note here that the first distal-end wires 25a₁ and the second distal-end wire 25a₂ may be combined into one wire that is wound around a pulley or the like.

Consequently, as the first distal-end wire 25a₁ is pulled, it causes the first distal-end trunk part 23a to rotate about the second distal-end shaft member 23d, as shown in Fig. 2(b), and as the second distal-end wire 25a₂ is pulled, it causes the first distal-end trunk part 23a to rotate about the second distal-end shaft member 23d in a direction opposite to that shown in Fig. 2(b). Such structure ensures that the first distal-end trunk part 23a of the distal-end action part 23 rotates about the first distal-end shaft member 23b and the second distal-end shaft member 23d with respect to the second distal-end trunk part 23c.

A relation of the third distal-end wire 25b₁ to the fourth distal-end wire 25b₂, because of being similar to the relation of the first distal-end wire 25a₁ to the second distal-end wire 25a₂, is not explained anymore.

The first insert part 22 is a tubular member that couples the distal-end part 21 to the distal-end action part 23, and the second insert part 24 is a tubular member that couples the distal-end action part 23 to the first turning part 31 of the turning part 3.

The structure of the insert part 2 is not necessarily limited to this structure; so it may be modified in various manners. For instance, a plurality of articulating pieces and a plurality of joint parts may alternately be combined so as to achieve a curving state having a higher degree of freedom. Preferably, multiple sets of articulating piece and joint parts are rotated and located for each 90° with the axis of the curving part extended in a straight line, that is, with the center axis C of the second insert part 24 as center, because three-dimensional movements are achievable. Note also that the distal-end action part 23 is not always limited to the universal joint; so it may have a bendable structure including a combined pitching joint and yawing joint.

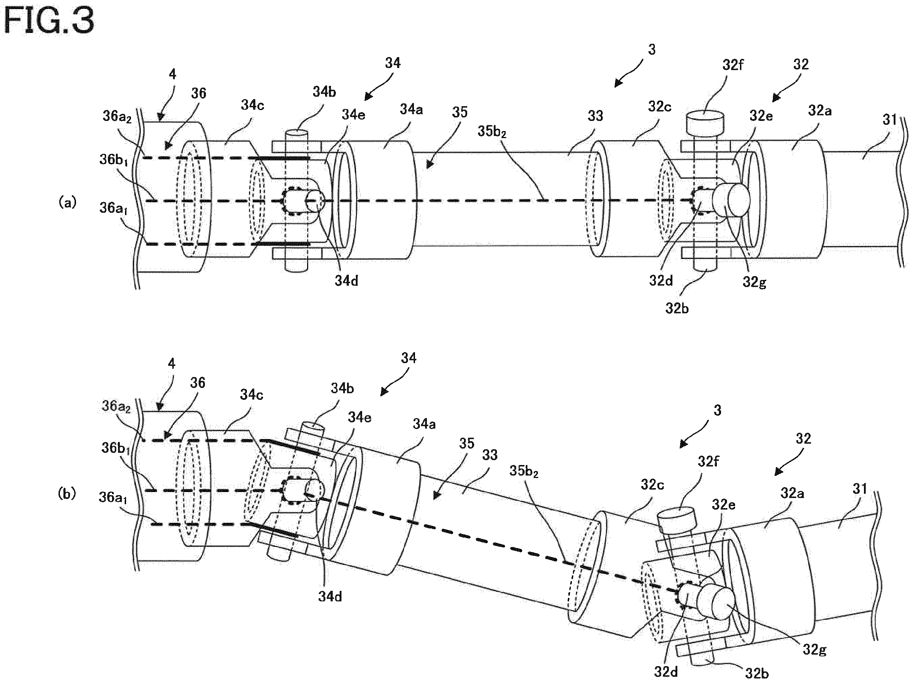

Fig. 3 is a schematic enlarged view of the turning part 3 according to the first embodiment: Fig. 3(a) is a schematic enlarged view of the turning part 3 in a normal linear state, and Fig. 3(b) is a schematic enlarged view of the turning part 3 in a bending state.

The passive part 32 is made up of a universal joint or the like. In the embodiment described herein, the passive part 32 has a structure in which a first passive shaft member 32b adapted to rotatably support a tubular first passive trunk part 23a coupled to a first turning part 31 and a second passive shaft member 32d adapted to rotatably support a tubular second passive trunk part 32c coupled to a second turning part 33 are attached to a tubular passive articulating piece 32e in a cross configuration.

The first passive shaft member 32b is provided with a first restriction part 32f, and the second passive shaft member 32d is provided with a second restriction part 32g. The first restriction part 32f and the second restriction part 32g are each made up of an electromagnetic clutch, an electro-magnetic brake or the like. The first restriction part 32f and the second restriction parts 32g may stop the rotation of the passive part 32 relative to the first passive shaft member 32b and the second passive shaft member 32d, respectively. In the embodiment described herein, the passive part 32 is actuated for rotation when the turning action part 34 turns.

The turning action part 34 is made up of a universal join or the like. In the embodiment described herein, the turning action part 34 has a structure wherein a first turning shaft member 34b adapted to rotatably support a tubular first turning trunk part 34a coupled to a second turning part 33 and a second turning shaft member 34d adapted to rotatably support a tubular second turning trunk part 34c coupled to the driver 4 are attached to a turning articulating piece 34e in a cross configuration.

The first turning shaft member 34b is provided on one side with a first turning wire 36a₁ and on the other side with a second turning wire 36a₂ with the turning articulating piece 34e in between, and the second turning shaft member 34d is provided on one side with a third turning wire 36b₁ and on the other side with a fourth turning wire 36b₂ (not shown) with the turning articulating piece 34e in between.

It is here to be noted that the turning action part 34 is not always limited to the universal joint; so it may have a turnable structure including a combined pitching joint and yawing joint.

The second turning part 33 is a tubular member that couples the passive part 32 to the turning action part 34.

It is here to be understood that the passive part 32 may otherwise be constructed if it is actuated in a direction opposite to the turning direction of the turning action part 34.

The first turning wire 36a₁ and the second turning wire 36a₂ are connected to a first turning driving unit such as an actuator (to be described later) of the driving unit 4 such that they can be pulled or let out. As the first turning wire 36a₁ is pulled toward the driver 4 side, it causes the second turning wire 36a₂ to be let out of the driver 4 side, and as the second turning wire 36a₂ is pulled toward the driver 4 side, it causes the first turning wire 36a₁ to be let out of the driver 4 side. Note here that the first turning wire 36a₁ and second turning wire 36a₂ may be combined into one wire that is wound around a pulley or the like.

As the first turning wire 36a₁ is pulled toward the driver 4 side, it causes one side of the first turning shaft member 34b of the turning action part 34 to be pulled toward the driver 4 side. At this time the second turning wire 36a₂ is let out of the driver 4 side.

Consequently, as the first turning wire 36a₁ is pulled, it causes the first turning trunk part 34a and the second turning part 33 to be rotated to one side of the first truing shaft member 34c with the second turning shaft member 34d as center. Upon such rotation of the first turning trunk part 34a, a part of the passive part 32 on the distal-end side of the first passive trunk part 32a is set in such a way as to be rotated toward the other side of the first passive shaft member 32b with the second passive shaft member 32d as center. In other words, the first turning part 31, second turning part 33 and driver 4 are located in a Z configuration.

As the second turning wire 36a₂ is pulled, it causes the first turning trunk part 34a and the first passive trunk part 32a to be rotated in the opposite direction. The relation of the third turning wire 36b₁ to the fourth turning wire 36b₂, because of being similar to the relation of the first turning wire 36a₁ to the second turning wire 36a₂, is not explained anymore.

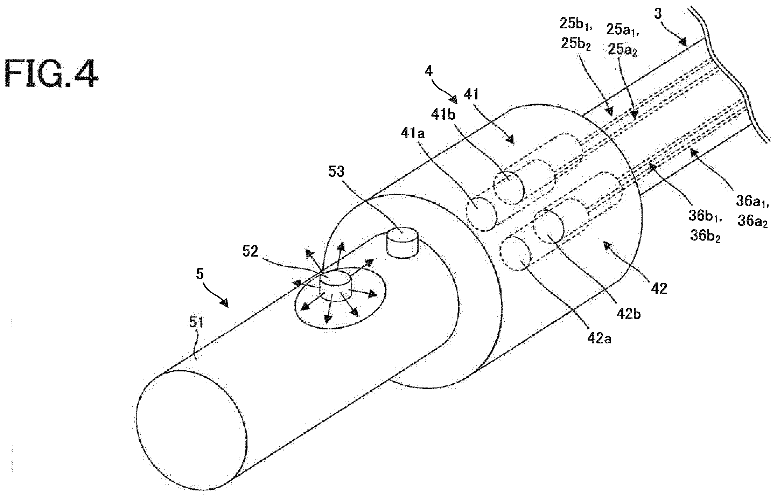

Fig. 4 is a schematic enlarged view of the driving unit 4 and operating unit 5 according to the first embodiment.

The driving unit 4 is located between the turning part 3 and the operating unit 5, and includes an actuator, etc. by which the wires can be pulled or let out. The driving unit 4 includes a distal-end driving unit 41 adapted to actuate a wire 25 connected to the distal-end action part 23 and a turning driving unit 42 adapted to actuate a wire 36 connected to the turning action part 34.

The distal-end driving unit 41 includes a first distal-end driving unit 41a adapted to enable the first distal-end wire 25a₁ and the second distal-end wire 25a₂ to be pulled and let out, and a second distal-end driving unit 41b adapted to enable the third 25b₁ and the fourth distal-end wire 25b₂ to be pulled and let out.

The turning driving unit 42 includes a first turning driving unit 42a adapted to enable the first turning wire 36a₁ and the second turning wire 36a₂ to be pulled and let out, and a second turning driving unit 42b adapted to enable the third turning wire 36b₁ and the fourth turning wire 36b₂ to be pulled and let out.

The operating unit 5 includes a grip 51 grasped by the operator, an operation instruction unit 52 including a joystick or the like for instructing the distal-end part 2 and turning part 3 to be put in the grip 51, and a mode switchover instruction unit 53 capable of being pressed in the grip 51.

While the grip 51 is shown to have a columnar shape in the embodiment described herein, it is to be appreciated that it may have any desired easy-to-grasp shape.

In response to the operation of the operation instruction unit 52, a protruding rod-like lever such as a joystick using a potentiometer is tilted down thereby bending the distal-end part 2 in the tilting direction and turning the turning part 3. Note here that a pointing device, a touch pad or the like may be used for the operation instruction unit 52.

The mode switchover instruction unit 53 is made up of a push switch or the like. With the push switch or the like being not depressed, usually, the mode switchover instruction unit 53 is set a manual mode as a first mode, and while the push switch or the like is pressed down, it provides an instruction about the switchover to an electrically operated mode as a second mode. Note here that a footswitch or the like may be used for the mode switchover instruction unit 53.

It is here to be noted that the driving unit 4 may be incorporated in the grip 51 of the operating unit 5 for integration of the driving unit 4 and operating unit 5. The actuator forming part of the driving unit 4 may use a motor or the like that makes it possible to take up or let out the wires by means of pulleys, etc.

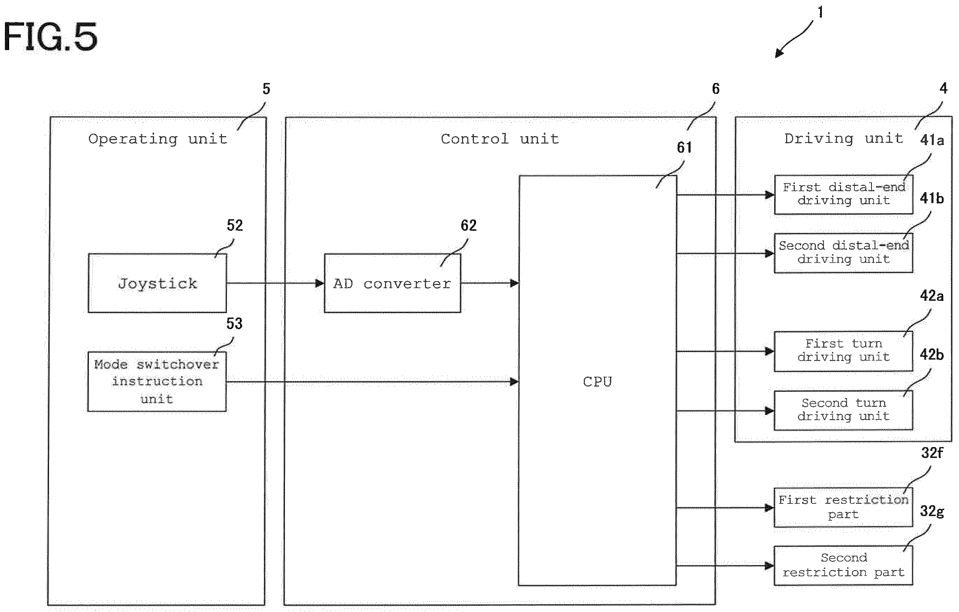

Fig. 5 is a block diagram for the manipulator 1 according to the first embodiment.

A signal entered from the joystick 52 of the operating unit 5 is entered in the CPU 61 of the control unit 6 by way of an AD converter 62. A signal for the switchover entered from the mode switchover instruction unit 53 for the switchover between the manual mode and the electrically operated mode is entered in the CPU 61.

In response to the signal entered from the operating unit 5, the CPU 61 of the control unit 6 controls the driving unit 4 and the restriction parts 32f, 32g.

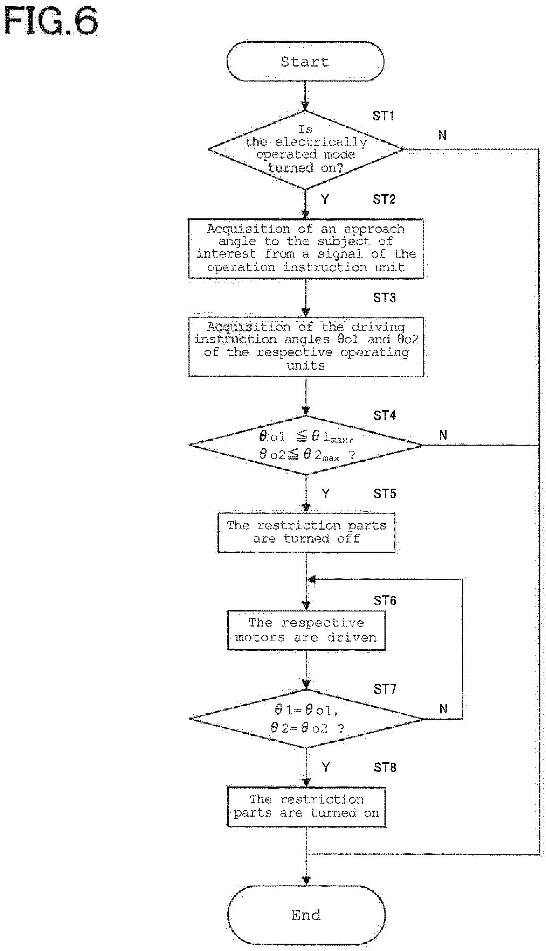

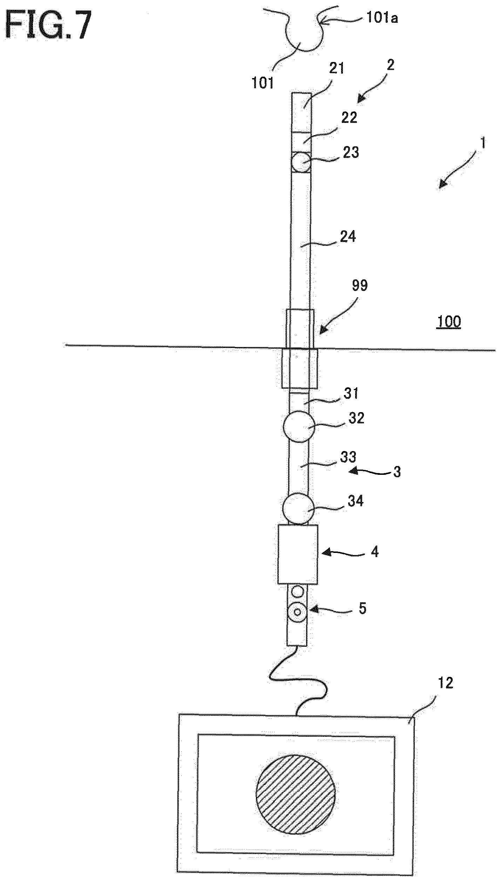

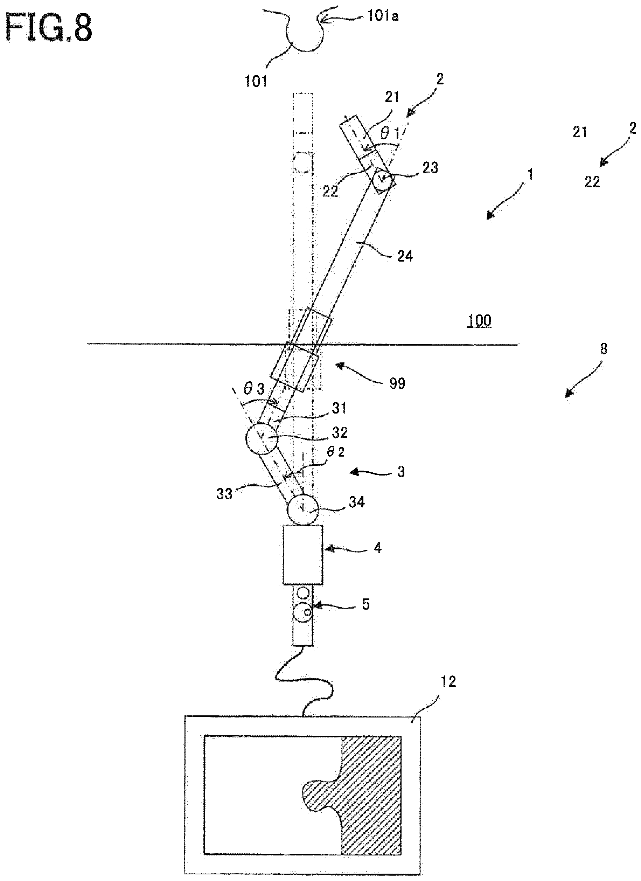

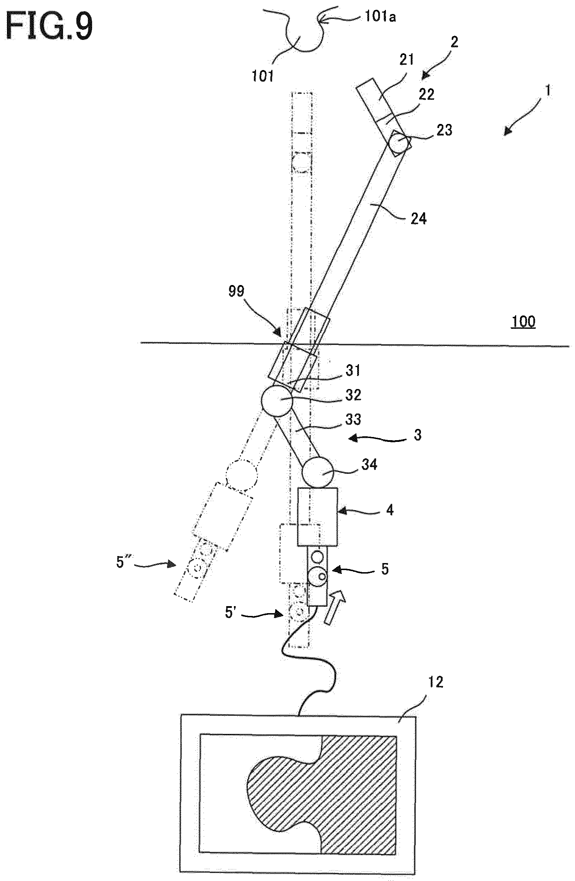

Fig. 6 is a control flowchart for the manipulator 1 according to the first embodiment, and Fig. 7 is illustrative in schematic of a state of the manipulator 1 according to the first embodiment before subjected to the electrically operated mode control. Fig. 8 is illustrative in schematic of a state of the manipulator 1 according to the first embodiment just after subjected to the electrically operated mode control, and Fig. 9 is illustrative in schematic of a state of the manipulator 1 according to the first embodiment upon a transition from after the electrically operated mode control to the manual mode.

While the manipulator 1 according to the first embodiment operates actually in a three-dimensional space, it is to be understood that for an easy understanding of operation, the embodiment described herein is explained with reference to an operation within a two-dimensional plane using the schematic views of Figs. 7, 8 and 9. The same will go for the following embodiments.

Now suppose that before control of the first embodiment, the insert part 2 of the manipulator 1 is already inserted through the body cavity 100 inside the skin, as shown in Fig. 7, and suppose that the endoscope in the manipulator 1 is taking images of a subject of interest 101. The electrically operated mode control gets started in response to an instruction from the mode switchover instruction unit 53.

First in Step 1, it is determined whether or not the electrically operated mode is turned on by the mode switchover instruction unit 53 (ST1).

When the electrically operated mode is turned off and instead the manual mode is turned on in Step 1, the electrically operated mode control gets done.

When the electrically operated mode is turned on in Step 1, the processing goes to Step 2 in which an approach angle to the subject of interest is acquired from a signal from the operation instruction unit 52 (ST2).

Then, the processing goes to Step 3 in which the first driving instruction angle θο1 of the distal-end action part 23 and the second driving instruction angle θο2 of the turning action part 34 are computed (ST3).

Then, the processing goes to Step 4 in which it is determined whether or not the driving instruction angles θo of the action parts 23 and 34 are within the driving ranges, respectively (ST4). When the driving instruction angles of the action parts 23 and 34 do not satisfy θo1, θo2≦maximum enabling angles θ1ₘₐₓ, θ2ₘₐₓ or, in another parlance, when at least one of the action parts 23 and 34 exceeds the enabling range, the electrically operated mode control gets done. Note here that at the time of completion of the electrically operated mode, it is preferable that a warning or the like that the electrically operated mode is out of control may be given to the operator, and that the processing may get done after the action parts 23 and 34 are driven up to the maximum enabling angles θ1_{max,} θ2ₘₐₓ.

In Step 4, when the driving instruction angles of the action parts 23 and 34 satisfy θo1, θo2≦maximum enabling angles θ1ₘₐₓ, θ2ₘₐₓ, the processing goes to Step 5 in which the restriction parts 32f and 32g are each turned off (ST5). With the restriction parts 32f and 32g turned off, the passive part 32 gets ready to rotate in association with the turning action part 34.

Then, the processing goes to Step 6 in which the first distal-end driving unit 41a, second distal-end driving unit 41b, first turning driving unit 42a and second turning driving unit 42b are driven (ST6).

Then, the processing goes to Step 7 in which it is determined whether or not the driving angles θ1 and θ2 are equal to the driving instruction angles θo1 and θo2, respectively (ST7).

When the driving angles θ1 and θ2 are found to be not equal to the driving instruction angles θo1 and θo2 in Step 7, the processing goes back to Step 6.

When the driving angles θ1 and θ2 are found to be equal to the driving instruction angles θo1 and θo2 in Step 7, the processing goes to Step 8 in which the restriction parts 32f and 32g are tuned on (ST8). With the restriction parts 32f and 32g turned on, the passive part 34 remains incapable of rotating with respect to the turning action part 34. Note here that the bending operation by the distal-end driving unit 41 and the turning operation by the turning driving unit 42 may be implemented in any desired sequence.

As a result of this electrically operated mode control, the manipulator 1 is actuated after the electrically operated mode control such that, as shown in Fig. 8, the distal-end action part 23 is rotated to make the bending angle θ1 equal to the first driving instruction angle θo1 and the turning action part 34 is rotated to make the bending angle θ2 equal to the second driving instruction angle θo2. In response to the bending of the distal-end action part 23 and turning action part 34, the passive part 32 is then rotated by the bending angle θ3.

In this state, an image of a first part 101a of the subject of interest 101 appears on the monitor 12, but that image will be nothing but the one coming from a remote position.

After that, the operator presses in the manipulator 1 so that, as shown in Fig. 9, the distal-end part 21 comes close to the subject of interest 101, resulting in appearance on the monitor 12 of the image of the first part 101a of the subject of interest 101 on a magnified scale.

Referring to the first embodiment, it is to be noted that as an example the operator presses in the manipulator 1 along the angle of a trocar 99. If the manipulator 1 includes none of the turning part 3 or if the manipulator 1 is operated by the operator in the manual mode with no actuation of the restriction parts 32f and 32g, a virtual operating unit 5" will come to a position as shown in Fig. 9.

Consequently, the operator may move the operating unit 5 just a bit from the operating unit 5' in the original position without changing the angle as compared with the virtual operating unit 5" whereby the distal-end part 21 moves to the desired position so as to display the desired image on the monitor 12.

When the operator sets free the electrically operated mode by the mode switchover instruction unit 53 after the electrically operated mode control, the manipulator 1 may be actuated in such a way as to allow the distal-end action part 23, passive part 32 and turning action part 34 to go back to the original straight line state. Alternatively, they may remain in situ at the time of setting free the electrically operated mode, and then go back to the original straight line state by means of other button. Then, pulling the manipulator 1 in the straight line state from within the body cavity 100 is all that is needed for the operator.

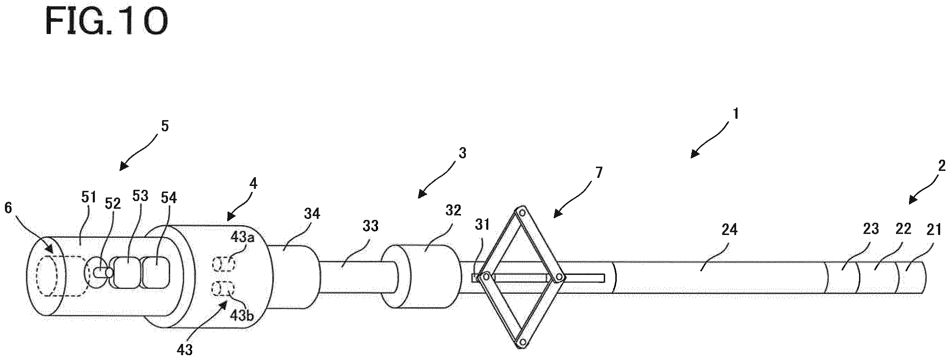

Fig. 10 is a schematic view of the manipulator 1 according to the second embodiment.

In the manipulator 1 according to the second embodiment, a distance setting unit 54, an extension/contraction driving unit 43 and a extension/contraction part 7 are added to the manipulator 1 according to the first embodiment.

The distance setting unit 54 is provided in the operating unit 5 to set instruction information about a distance between the distal-end part 21 and the subject of interest according to preset distance stage. Each time such a button on the distance setting unit 54 as shown in Fig. 10 is depressed down, for instance, the distance instruction information may be selected from a plurality of preset distance stages such as first, second and third distances.

The extension/contraction driving unit 43 is an actuator that drives the extension/contraction part 7 and is preferably placed in the driving unit 4 or the operating unit 5. In the second embodiment, there are two driving units 43 provided: an extension driving unit 43a and a contraction driving unit 43b. However, it is not always necessary to provide two such extension/contraction driving units 43; one or three or more extension/contraction driving units 43 may be provided as required.

It is here to be appreciated that the structure of the extension/contraction driving unit 43 including a motor, a pulley and so on may be modified in various manners. For instance, while the extension/contraction driving unit 43 is shown to be placed in the driving unit 4 in Fig. 10, it is to be understood that it may be provided separately from the driving unit 4.

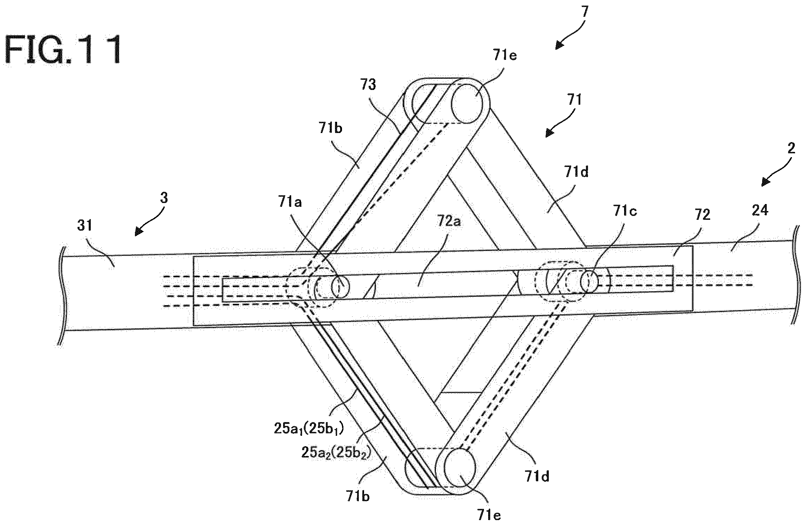

Fig. 11 is a schematic enlarged view of the extension/contraction part 7 according to the second embodiment.

The extension/contraction part 7 includes a link part 71 coupled on one side to the turning part 3 and on the other side to the insert part 2, and a guide part 72 that guides the actuation of the link part 71.

The link part 71 includes a first protrusion 71a attached to the first turning part 31 and guided by the guide part 72, a first arm 71b rotatably attached to the first protrusion 71a, a second protrusion 71c attached to the second insert part 24 and guided by the guide part 72, a second arm 71d rotatably attached to the second protrusion 71c, and a connector 71e adapted to make a connection between the first 71b and the second arm 71d.

The guide part 72 is an elongate member that is fixed on one side to the first turning part 31, and provided with an elongate slot 72a into which the first 71a and the second protrusion 71c are inserted; in other words, the first 71a and the second protrusion 71c are movable along the elongate slot 72a. Note here that the guide part 72 may be fixed on either one of both sides in place. For instance, the guide part 72 may be fixed on the other side rather than one side to the second insert part 24, contrary to the embodiment described herein.

In the second embodiment described herein, the first arm 71b, the second arm 71d and the connector 71e are provided in twos with the guide part 72 in between. At least one connector 71e is provided around it with a linking wire 73 as an extension/contraction power transmission part, and the linking wire 73 is taken up or let out by the driving unit 4 shown in Fig. 10 thereby causing extension and contraction of the link part 71 having a so-called pantograph structure.

Inside the first 71b, and the second arm 71d there is a cavity through which wirings or the like are passed for the first distal-end wire 25a₁, second distal-end wire 25a₂, third distal-end wire 25b₁, fourth distal-end wire 25b₂ (not shown), linking wire 73 and electric parts.

It is here to be noted that there may be multiple link parts 71 and guide parts 72 provided so as to make the advanceable/retractable movement distance long.

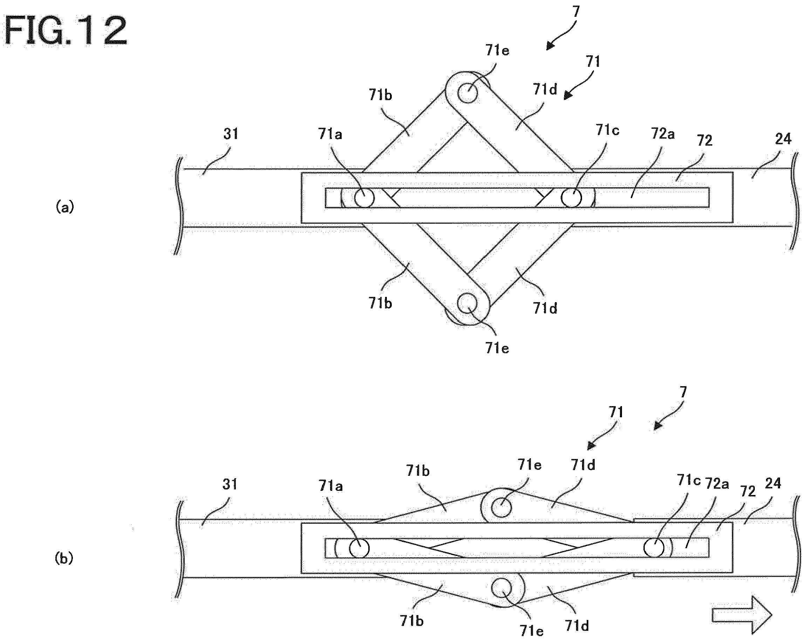

Fig. 12 is a schematic view of one example of operation of the extension/contraction part 7 in the second embodiment: Fig. 12(a) is a schematic view of the extension/contraction part 7 in a contracting state and Fig. 12(b) is a schematic view of the extension/contraction part 7 in an extending state.

In the second embodiment, the pantograph mechanism is applied as an example of the extension/contraction part 7 that generates advanceable/retractable movement of the insert part 2 in the manipulator 1.

As can be seen from Fig. 11, the linking wire 73 is wound around the connector 71e of the link part 71 that is actuated by the extension/contraction driving unit 43 adapted to take up and let out the linking wire 73.

As the extension/contraction part 7 in the state shown in Fig. 12(a) is driven by the extension/contraction driving unit 43 to take up the linking wire 73, it causes the connector 71e to be pulled by the linking wire 73 so that the first arm 71b rotates about the first protrusion 71a, whereupon the second arm 71d having the link formed rotates about the second protrusion 71c.

Consequently, the angle that the first arm 71b forms with the second arm 71d grows large as shown in Fig. 12(b), causing movement of the second protrusion 71c away from the first protrusion 71a along the elongate slot 72a in the guide part 72. Accordingly, the distance between the first 71a and the second protrusion 71c grows long, thus leading to an elongation of the extension/contraction part 7.

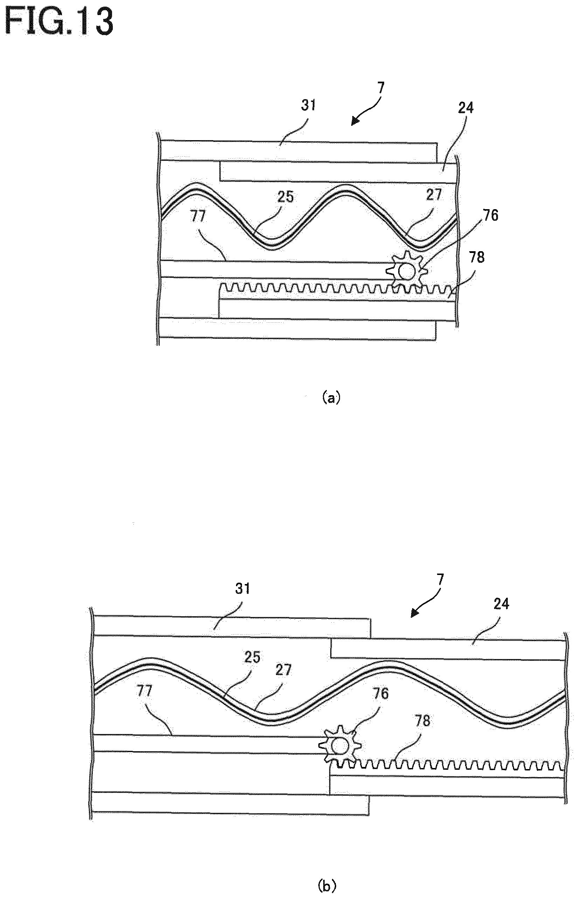

Fig. 13 is a schematic view of another example of the extension/contraction part 7: Fig. 13(a) is a schematic view of the extension/contraction part 7 in a contracting state, and Fig. 13(b) is a schematic view of the extension/contraction part 7 in an extending state.

In the extension/contraction part 7 shown in Fig. 13, there is a gear mechanism interposed between the insert part 2 and the turning part 3 as an example of the mechanism for generating extension/contraction movement of the insert part 2 in the manipulator 1. The extension/contraction part 7 includes a pinion 76, a pinion driving wire 77 serving as an extension/contraction driving power transmission, and a rack 78.

The pinion 76 is driven by transmission of the driving force of the extension/contraction driving unit 43 by the pinion driving wire 77. Following the rotation of the pinion 76, the second insert part 24 provided with the rack 78 moves relative to the first turning part 31 for extension and contraction of the manipulator 1.

In the state of Fig. 13(a), the second insert part 24 is retracted into the first turning part 31 so that the exposed part of the second insert part 24 is reduced in length. As the extension/contract driving unit 43 shown in Fig. 10 is actuated from this state, it causes the pinion 76 to rotate so much so that, as shown in Fig. 13(b), the rack 78 moves whereby the second insert part 24 moves forward.

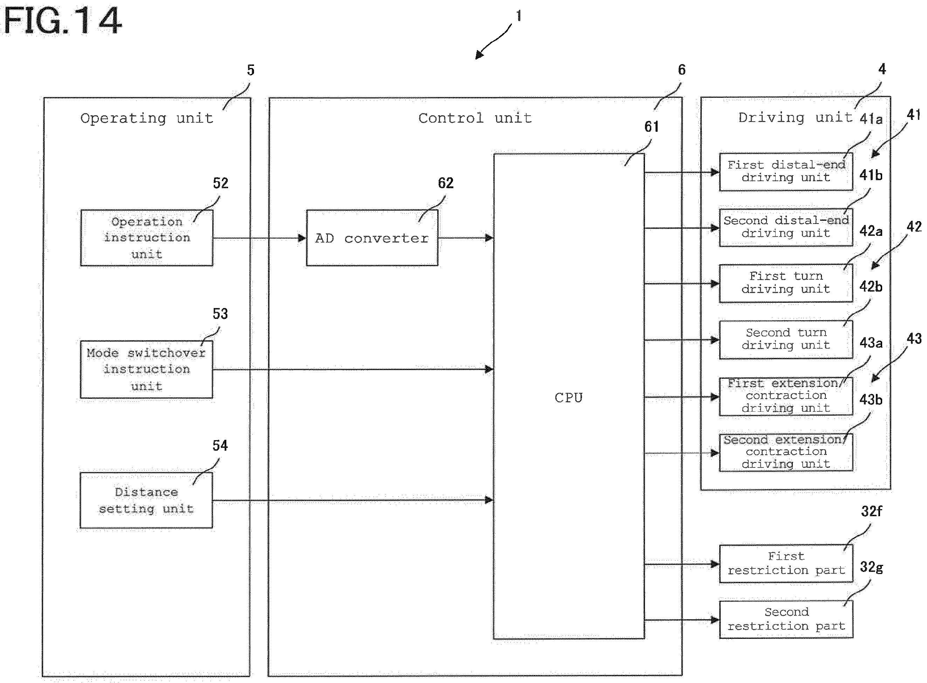

Fig. 14 is a block diagram for the manipulator 1 according to the second embodiment.

The operating unit 5 according to the second embodiment includes the distance setting unit 54 for determining the desired distance between the distal-end part 21 and the subject of interest, as shown in Fig. 10, in addition to the operating unit 5 in the first embodiment. A distance setting signal entered from the distance setting unit 54 is entered in CPU 61.

In response to the signal entered from the operating unit 5 including the distance setting unit 54, the CPU 61 in the control unit 6 controls the extension/contraction driving unit 43.

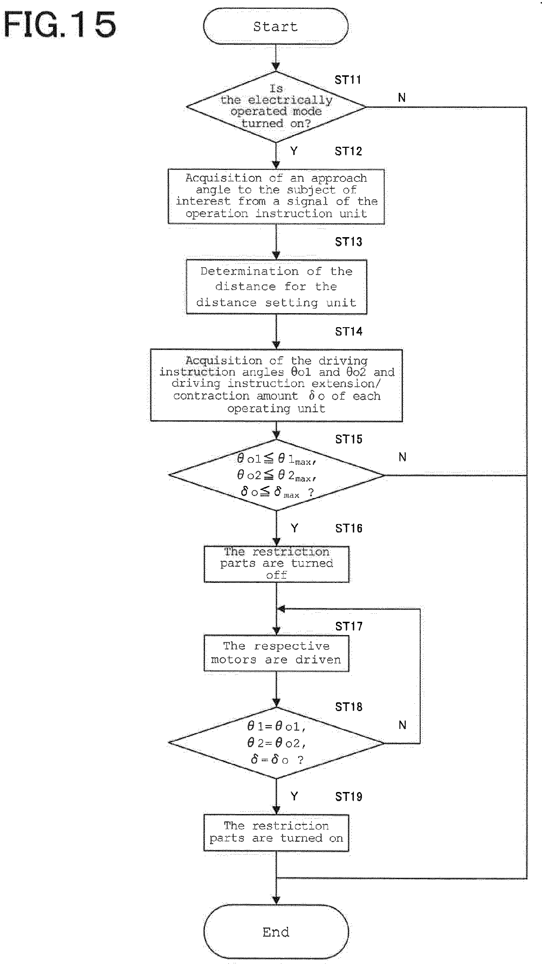

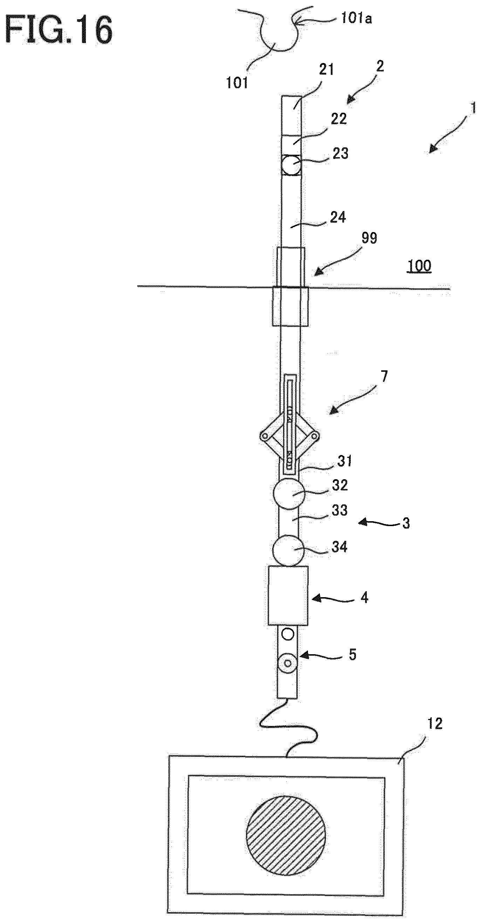

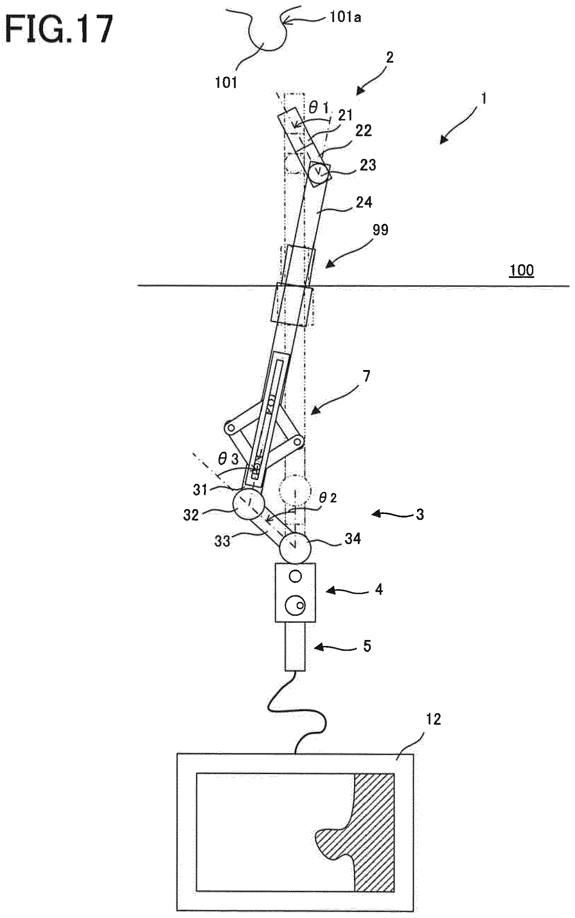

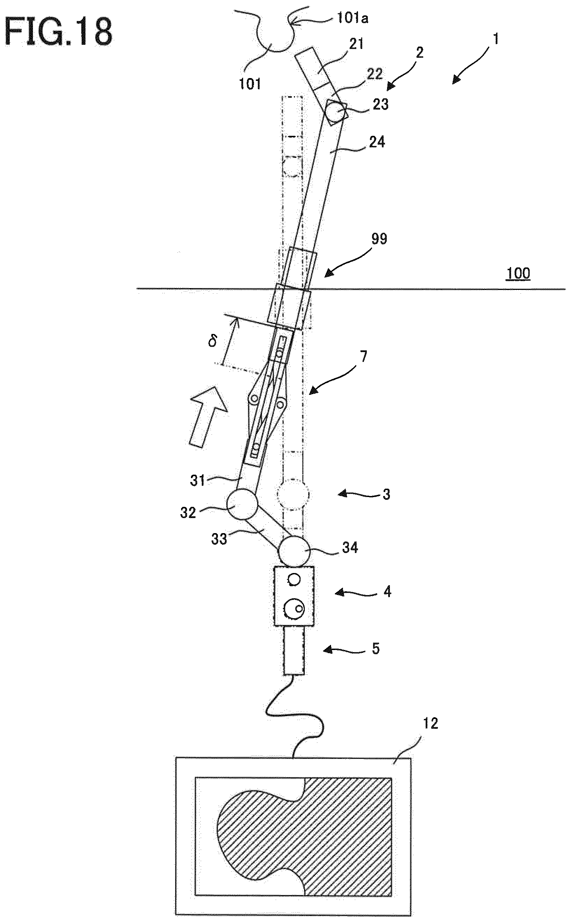

Fig. 15 is a control flowchart for the manipulator 1 according to the second embodiment. Fig. 16 is a schematic view of a state of the manipulator 1 according to the second embodiment before subjected to the electrically operated mode control. Fig. 17 is a schematic view of a virtual state of the manipulator 1 according to the second embodiment after control of the turning driving unit in the electrically operated mode. Fig. 18 is a schematic view of a state of the manipulator 1 according to the second embodiment after control of the extension/contraction driving unit in the electrically operated mode.

Now suppose that before control according to the second embodiment, the insert part 2 of the manipulator 1 is already inserted through the body cavity 100 inside the skin, as shown in Fig. 16, and that the endoscope in the manipulator 1 is taking images of a subject of interest 101. The electrically operated mode control gets started in response to an instruction from the mode switchover instruction unit 53.

First in Step 11, it is determined whether or not the electrically operated mode is turned on by the mode switchover instruction unit 53 (ST11).

When the electrically operated mode is found to be turned off and instead the manual mode is held on in Step 11, the electrically operated mode control gets done.

When the electrically operated mode is turned on in Step 11, the processing goes to Step 12 in which an approach angle to the subject of interest is acquired from a signal from the operation instruction unit 52 (ST12).

Then, the processing goes to Step 13 in which the distance is set for the distance setting unit 54 (ST13).

Then, the processing goes to Step 14 in which the approach angle to the subject of interest is computed from a signal from the operation instruction unit 52, and the first driving instruction angle θo1 of the distal-end action part 23, the second driving instruction angle θo2 of the turning action part 34 and the driving instruction extension/contraction amount δo are computed from the distance set at the distance setting unit 54 (ST14).

Then, the processing goes to Step 15 in which it is determined whether or not the driving instruction angles θo and extension/contraction amount δo of the action parts 23 and 34 are within the acceptable ranges, respectively (ST15). In Step 15, when the driving instruction angles of the action parts 23 and 34 do not satisfy θo1, θο2≦maximum enabling angles θ1ₘₐₓ, θ2ₘₐₓ or, in another parlance, when at least one of the action parts 23 and 34 exceeds the enabling range, or when the driving instruction extension/contraction amount for the extension/contraction part 7 does not meet δο≦maximum enabling amount δₘₐₓ or, in another parlance, when the extension/contraction part 7 exceeds the extension/contraction enabling range, the electrically operated mode control gets done. Note here that at the time of completion of the electrically operated mode, it is preferable that a warning or the like that the electrically operated mode goes beyond the controllable range may be given to the operator, and that the action parts 23 and 34 may be driven to the maximum enabling angles θ1ₘₐₓ, θ2ₘₐₓ to extend and contract the extension/contraction part 7 to the maximum enabling range δₘₐₓ.

In Step 15, when the driving instruction angles θο1 and θo2 of the action parts 23 and 34 satisfy θo1, θo2≦ maximum enabling angles θ1max, θ2ₘₐₓ and when the driving instruction extension/ contraction amount for the extension/contraction part 7 meets δο≦maximum enabling amount δ max, the processing goes to Step 16 in which the restriction parts 32f and 32g are each turned off (ST16). With the restriction parts 32f and 32g turned off, the passive part 32 gets ready to rotate in association with the turning action part 34.

Then, the processing goes to Step 17 in which the first distal-end driving unit 41, turning driving unit 42 and extension/contraction driving unit 43 are driven (ST17).

Then, the processing goes to Step 18 in which it is determined whether or not the driving angles θ1 and θ2 and extension/contraction amount δ are equal to the driving instruction angles θο1 and θo2 and driving instruction extension/contraction amount δo (ST18).

When the driving angles θ₁ and θ₂ and extension/contraction amount δ are found to be not equal to the driving instruction angles θo1 and θo2 and driving instruction expansion amount δo in Step 18, the processing goes back to Step 17.

In Step 18, when the driving angles θ₁ and θ₂ and extension/contraction amount δ are found to be equal to the driving instruction angles θο1 and θo2 and driving instruction extension/contraction amount δo, the processing goes to Step 19 in which the restriction parts 32f and 32g are tuned on (ST19). With the restriction parts 32f and 32g turned on, the passive part 32 remains incapable of rotating with respect to the turning action part 34. Note here that the bending operation by the distal-end driving unit 41, the turning operation by the turning driving unit 42 and extension/contraction operation of the extension/contraction part 7 may be implemented in any desired sequence.

As a result of this electrically operated mode control, the manipulator 1 is actuated after the electrically operated mode control such that, as shown in Fig. 17, the distal-end action part 23 is rotated to make the first bending angle θ1 equal to the first driving instruction angle θο1 and the turning action part 34 is rotated to make the second bending angle θ2 equal to the second driving instruction angle θο2. However, this just only leads to a virtual state where in response to the bending of the distal-end action part 23 and turning action part 34, the passive part 32 is rotated by the third bending angle θ3; even if the image of the first part 101a of the subject of interest 101 appears on the monitor 12, that image will be nothing but the one coming from a remote position, as shown in Fig. 17.

For this reason, the extension/contraction part 7 is extended to make the extension/contraction amount δ equal to the driving instruction extension amount δo, as shown in Fig. 18. Consequently, the image of the first part 101a of the subject of interest 101 appears on the monitor 12 on an enlarged scale. As shown in Fig. 18, therefore, the operator will be able to move the distal-end part 21 to the desired position with no substantial change in the original position of the operating unit 5 to show the desired image on the monitor 12; in actual movement, the manipulator 1 shifts from the state of Fig. 16 to the state of Fig. 18 in one stroke, not by way of the virtual state of Fig. 17.

When the operator sets free the electrically operated mode by the mode switchover instruction unit 53 after the electrically operated mode control, the manipulator 1 may be actuated such that the distal-end action part 23, passive part 32 and turning action part 34 to go back to the original straight line state. Alternatively, they may remain in situ at the time of setting free the electrically operated mode, and then go back to the original straight line state by means of other button. Then, pulling the manipulator 1 in the straight line state from within the body cavity 100 is all that is needed for the operator.

The first and second bending angles θ1 and θ2 and extension/contraction amount δ of the manipulator 1 in the electrically operated mode are now explained.

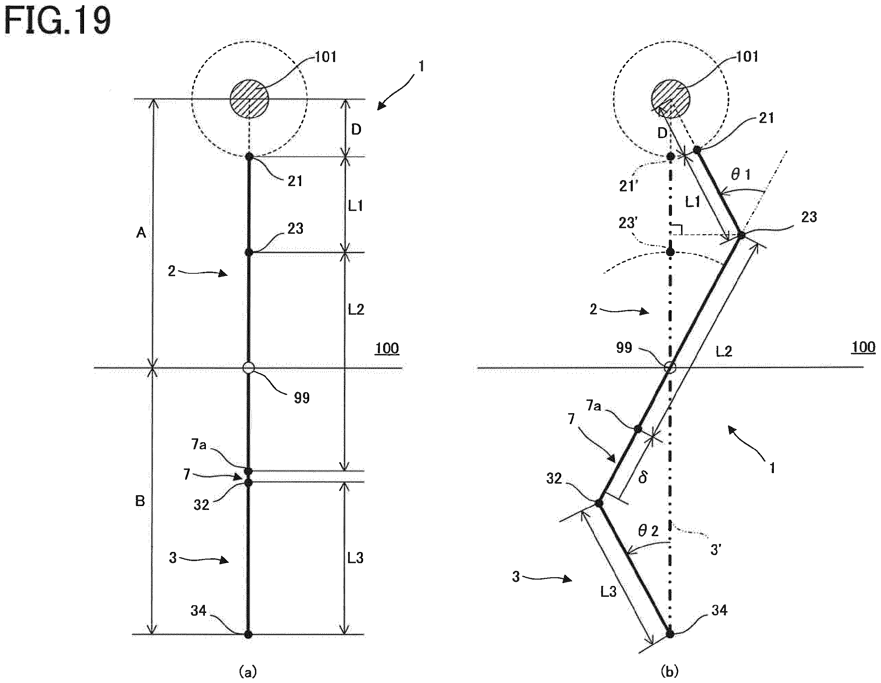

Fig. 19 is a schematic view of the geometrical configuration used for the electrically operated mode control of the manipulator 1 according to the second embodiment: Fig. 19(a) is a schematic view of the manipulator 1 according to the second embodiment before subjected to the electrically operated mode control, and Fig. 19(b) is a schematic view of the manipulator 1 according to the second embodiment after subjected to the electrically operated mode control.

In Fig. 19, the distal-end part 21 is indicated by a point 21 positioned at the most distal end, the distal-end action part 23 by a point 23, the trocar 99 by a point 99, the extension/contraction part 7 by a point 7, the passive part 32 by a point 32, and the turning action part 34 by a point 34.

The manipulator 1 according to the second embodiment is controlled in the electrically operated mode such that the distance between the post-control distal-end part 21 and the subject of interest 101 becomes equal to the value set at the distance setting unit 54 from the before-control state shown in Fig. 19(a) and the distal-end part 21 directs to the subject of interest 101.

In Fig. 19(b), the manipulator 1 before subjected to control is indicated by a phantom line. Here let D stand for the distance from the subject of interest 101 to the distal-end part 21, L1 stand for the distance from the distal-end part 21 to the distal-end action part 22, L2 stand for the distance from the distal-end action part 22 to the mount position of the extension/contraction part 7 on the insert part 2 side, and L3 stand for the distance from the passive part 32 to the turning action part 34. Here again let A stand for the distance from the subject of interest 101 to the position of the trocar 99 and B stand for the distance from the position of the trocar 99 to the turning action part 34.

With such a geometrical relationship, as an instruction signal about the approach angle is entered from the operation instruction unit 52, the control unit 6 is actuated to compute the first bending angle θ1 of the distal-end action part 23, the second bending angle θ2 of the turning action part 34 and the extension/ contraction amount δ of the extension/contraction part 7. In this case, the position of the turning action part 34 remains invariable so that there can be an approach taken to the subject of interest 101 from a varying angle without movement of the operating unit 5.

In one variation, as the instruction signal about the approach angle is entered from the operation instruction unit 52, the control unit 6 is actuated to compute the first bending angle θ1 of the distal-end action part 23, the second bending angle θ2 of the turning action part 34 and the extension/contraction amount δ of the extension/contraction part 7 by deriving a solution of inverse kinematics computation with respect to the respective joints and links while taking into consideration the rolling, pitching and yawing postures of the distal-end part 21. In the second embodiment, while computation is implemented while the distance D between the distal-end part 21 and the subject of interest 101 is kept constant, it is to be understood that computation may be implemented on the assumption that there is the subject of interest 101 present on an extension of the distal-end part 21. In other words, the distal-end part 21 may direct to the subject of interest 101 and the line of sight of the scope may direct to the subject of interest 101.

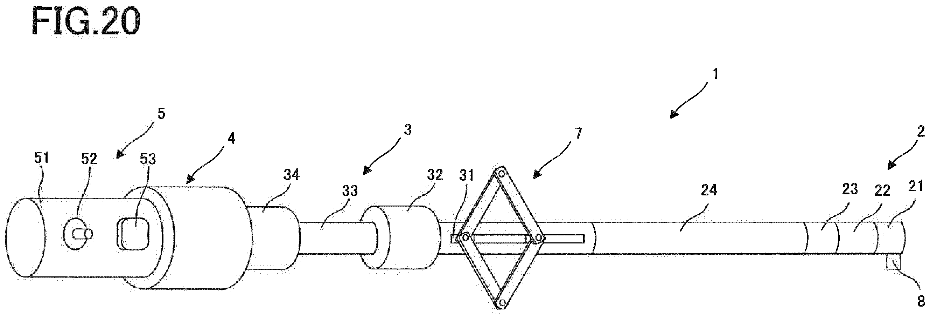

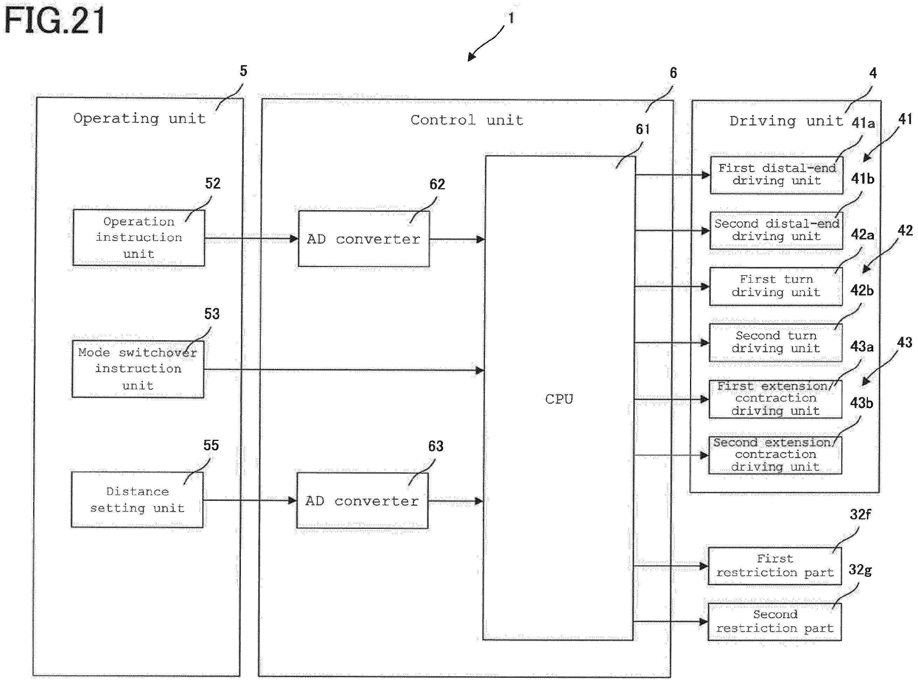

Fig. 20 is a schematic view of the manipulator 1 according to the third embodiment, and Fig. 21 is a block diagram for the manipulator 1 according to the third embodiment.

In the manipulator 1 according to the third embodiment, a distance measurement unit 8 is added to the manipulator 1 according to the second embodiment. As shown in Fig. 20, the distance measurement unit 8 is a sensor that is mounted on the distal-end part 21 to measure the distance between the distal-end part 21 and the subject of interest. As can be seen from Fig. 21, distance information measured by the distance measurement unit 8 is entered in the CPU 61 by way of an AD converter 63. In response to a signal entered from the operating unit 5 including the distance measurement unit 55, the control unit 6 controls the driving unit 4 and the respective restriction parts 32f and 32g.

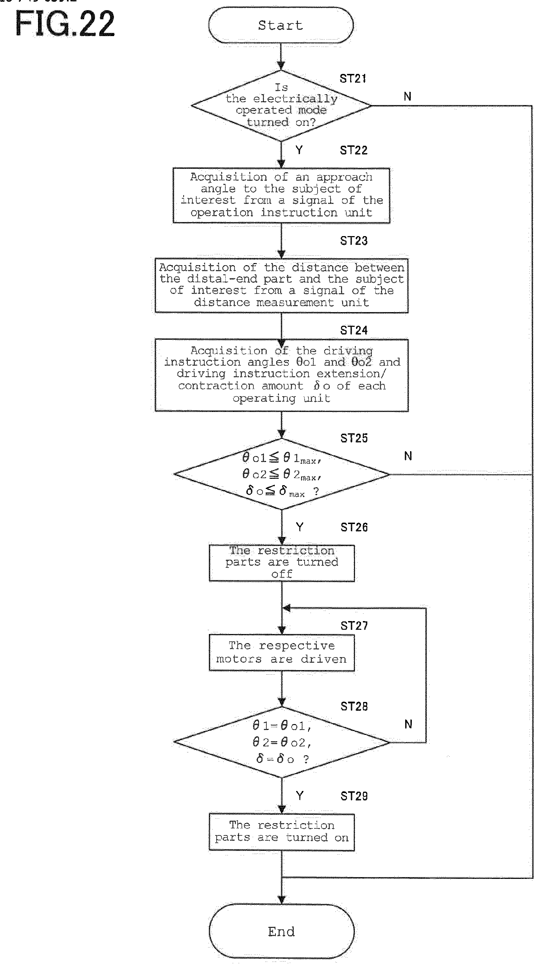

Fig. 22 is a control flowchart for the manipulator 1 according to the third embodiment.

First in Step 21, it is determined whether or not the electrically operated mode is turned on by the mode switchover instruction unit 53 (ST21).

When the electrically operated mode is turned off and instead the manual mode is held on in Step 21, the electrically operated mode control gets done.

When the electrically operated mode is turned on in Step 21, the processing goes to Step 22 in which an approach angle to the subject of interest is acquired from a signal from the operation instruction unit 52 (ST22).

Then, the processing goes to Step 23 in which the distance from the distal-end part 21 to the subject of interest is measured and acquired by the distance measurement unit 8 (ST23).

Then, the processing goes to Step 24 in which an approach angle to the subject of interest is computed from a signal from the operation instruction unit 52, and the first driving instruction angle θo1 of the distal-end action part 23, the second driving instruction angle θo2 of the turning action part 34 and the driving instruction extension/contraction amount δo are computed from the distance obtained by measurement by the distance measurement unit 8 (ST24).

Then, the processing goes to Step 25 in which it is determined whether or not the driving instruction angles θo and extension/contraction amount θo of the action parts 23 and 34 are within the acceptable ranges, respectively (ST25). In Step 25, when the driving instruction angles θo of the action parts 23 and 34 do not satisfy θo1, θo2≦maximum enabling angles θ1ₘₐₓ, θ2ₘₐₓ or, in another parlance, when at least one of the action parts 23 and 34 exceeds the enabling range or, alternatively, when the driving instruction extension/contraction amount for the extension/contraction part 7 does not meet δo≦maximum enabling amount δₘₐₓ or, in another parlance, when the extension/contraction part 7 goes beyond the extension/contraction enabling range, the electrically operated mode control gets done. Note here that at the time of completion of the electrically operated mode, it is preferable that a warning or the like that the electrically operated mode goes beyond the controllable range may be given to the operator, and that the action parts 23 and 34 may be driven to the maximum enabling angles θ1max, θ2ₘₐₓ to extend and contract the extension/contraction part 7 to the maximum enabling range δₘₐₓ.

In Step 25, when the driving instruction angles θo1 and θo2 of the action parts 23 and 34 satisfy θο1, θo2≦ maximum driving angles θ1ₘₐₓ, θ2ₘₐₓ and when the driving instruction extension/ contraction amount for the extension/contraction part 7 meets δo≦maximum enabling amount δₘₐₓ, the processing goes to Step 26 in which the restriction parts 32f and 32g are each turned off (ST26). With the restriction parts 32f and 32g turned off, the passive part 32 gets ready to rotate in association with the turning action part 34.

Then, the processing goes to Step 27 in which the distal-end driving unit 41, turning driving unit 42 and extension/contraction driving unit 43 are driven (ST27).

Then, the processing goes to Step 28 in which it is determined whether or not the driving angles θ1 and θ2 and extension/contraction amount δ are equal to the driving instruction angles θο1 and θo2 and driving instruction extension/contraction amount δo(ST28).

In Step 28, when the driving angles θ1 and θ2 and extension/contraction amount δ are found to be not equal to the driving instruction angles θo1 and θo2 and driving instruction extension/contraction amount δο, the processing goes back to Step 27.

In Step 28, when the driving angles θ1 and θ2 and extension/contraction amount δ are found to be equal to the driving instruction angles θο1 and θo2 and driving instruction extension/contraction amount δο, the processing goes to Step 29 in which the restriction parts 32f and 32g are tuned on (ST29). With the restriction parts 32f and 32g turned on, the passive part 32 remains incapable of rotating with respect to the turning action part 34. Note here that the bending operation by the distal-end driving unit 41, the turning operation by the turning driving unit 42 and extension/contraction operation of the extension/contraction part 7 may be implemented in any desired sequence.

As a result of this electrically operated mode control, the manipulator 1 is actuated after the electrically operated mode control such that, as shown in Fig. 17, the distal-end action part 23 is rotated to make the first bending angle θ1 equal to the first driving instruction angle θο1 and the turning action part 34 is rotated to make the second bending angle θ2 equal to the second driving instruction angle θo2. However, this just only gives rise to a virtual state where in response to the bending of the distal-end action part 23 and turning action part 34, the passive part 32 is rotated by the third bending angle θ3; even if the image of the first part 101a of the subject of interest 101 appears on the monitor 12, that image will be nothing but the one coming from a remote position, as shown in Fig. 17.

For this reason, the extension/contraction part 7 is extended to make the extension/contraction amount δ equal to the driving instruction extension amount δο, as shown in Fig. 18. Consequently, the image of the first part 101a of the subject of interest 101 appears on the monitor 12 on an enlarged scale. As shown in Fig. 18, therefore, the operator will be able to move the distal-end part 21 to the desired position with no substantial change in the original position of the operating unit 5 to show the desired image on the monitor 12. In actual movement, the manipulator 1 shifts from the state of Fig. 16 to the state of Fig. 18 in one stroke, not by way of the virtual state of Fig. 17.

When the operator sets free the electrically operated mode by the mode switchover instruction unit 53 after the electrically operated mode control, the manipulator 1 may be actuated such that the distal-end action part 23, passive part 32 and turning action part 34 go back to the original straight line state. Alternatively, they may remain in situ at the time of setting free the electrically operated mode, and then go back to the original straight line state by means of other button. Further, when the distance from the distal-end part 21 to the subject of interest as measured by the distance measurement unit 8 is longer than the predetermined distance, they may go back to the straight line state. Then, pulling the manipulator 1 in the straight line state from within the body cavity 100 is all that is needed for the operator.

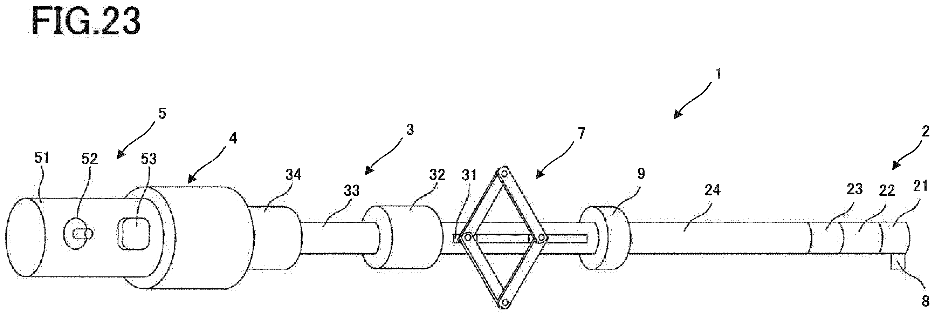

Fig. 23 is a schematic view of another example of the manipulator 1.

In this example, the insert part 2 is provided with a stopper 9. Having a diameter larger than that of the second insert part 24, the stopper 9 is provided at the end of the second insert part 24. The stopper 9 has a diameter larger than that of a through-hole in the trocar 99, so that it cannot extend through the trocar 99. It is thus possible to prevent the extension/contraction part 7 and turning part 3 from being accidentally inserted through the interior of the body.

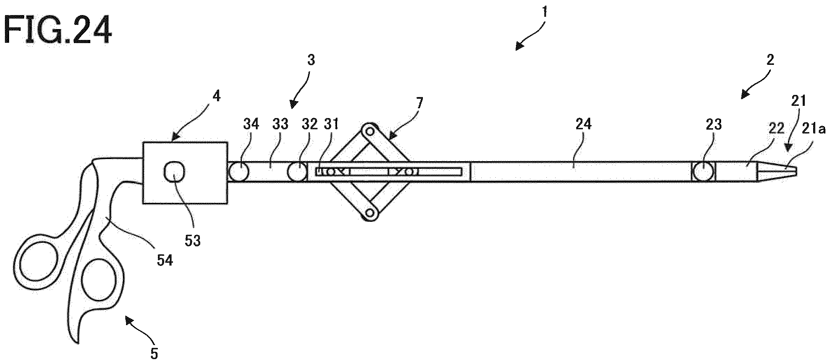

Fig. 24 is a schematic view of yet another example of the manipulator 1.

In this example, the distal-end part 21 is provided with a gripper part 21a serving as a treatment tool. The gripper part 21a is a pair of forceps or the like for taking hold of the subject of interest and cutting it. The gripper part 21a is opened or closed by operating wires (not shown) or the like by the operating unit 5 like scissors, and the manipulator 1 according to this example has otherwise the same structure as the manipulator 1 including an endoscope.

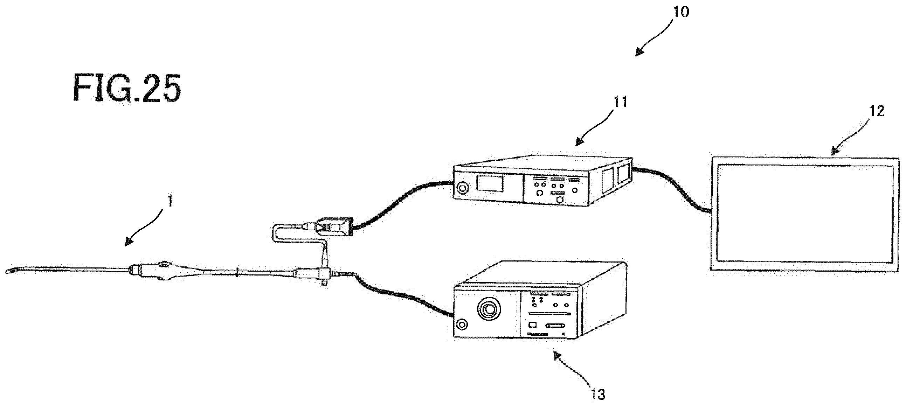

Fig. 25 is a schematic view of the manipulator system 10 according to one embodiment.

The manipulator system 10 according to the embodiment described herein includes the manipulator 1 including an endoscope, an image processor 11, a monitor 12, and a light source device 13.

The image processor 11 applies a variety of imaging processing such as Y adjustment, edge enhancement and output format transformation to an image signal obtained from the endoscope in the manipulator 1 to deliver an image signal to the monitor 12. The monitor 12 is provided to show an image signal received from the image processor 11 as a viewing image, and the light source device 13 emits illumination light out of the distal-end part 21 through a light guide fiber (not shown) in the manipulator 1 to light the subject of interest.

While the respective embodiments have been explained typically with reference to the manipulator integral with the operating unit, it is to be understood that the embodiment described herein may also be applied to a so-called master/slave system including a separate operating unit. In this case, the master provides a part of the operating unit, and the manipulator body including an insert part, a turning part and a driving unit is attached to a robot arm as the slave for remote operation. Note here that the driving unit may be positioned on the robot arm side.

It is here to be appreciated that the invention is in no sense limited to such embodiments as described above. While the explanation of some embodiments embraces numerous specific details for illustration, it would be obvious to those skilled in the art that diverse variations or modifications made thereto are included within the scope of the invention. In other words, illustrative embodiments of the invention are described without excluding generality from the claimed inventions and imposing any limitation thereon.

### Reference Signs List

- 1:: Manipulator
- 2:: Insert part
- 21:: Distal-end part
- 22:: First insert part
- 23:: Distal-end action part
- 24:: Second insert part
- 3:: Turning part
- 31:: First turning part
- 32:: Passive part
- 33:: Second turning part
- 34:: Turning action part
- 4:: Driving unit
- 41:: Distal-end driving unit
- 42:: Turning driving unit
- 43:: Extension/contraction driving unit
- 5:: Operating unit
- 51:: Grip
- 52:: Operation instruction unit
- 53:: Mode switchover instruction unit
- 6:: Control unit
- 7:: Extension/contraction part
- 71:: Link part
- 72:: Guide part
- 8:: Distance measurement unit
- 9:: Stopper
- 10:: Manipulator system
- 11:: Image processor
- 12:: Monitor
- 13:: Lighting device
- 99:: Trocar
- 100:: The body cavity
- 101:: Subject of interest

## Claims

1. A manipulator, **characterized by** comprising:
an insert part capable of being inserted into the body cavity;
a turning part for turning the insert part;
an operating unit for operating the insert part and the turning part;
a driving unit for driving the insert part and the turning part;
and
a control unit for controlling the driving unit in association with operation of the operating unit,
wherein
the insert part includes a bendable distal-end action part,
the turning part includes a turning action part for turning the insert part, and a passive part that is placed into passive action in association with turning action of the turning action part,
the driving unit includes a distal-end driving unit for generating a driving force adapted to bend the distal-end action part and a turning driving unit for generating a driving force adapted to turn the turning action part, and
the control unit controls the distal-end driving unit and the turning driving unit in association with operation of the operating unit.

2. A manipulator according to claim 1, further comprising
a extension/contraction part for extending and contracting the insert part,
wherein
the driving unit includes an extension/contraction driving unit for generating a driving force adapted to extend and contract the extension/contraction part, and
the control unit controls the extension/contraction driving unit in association with operation of the operating unit.

3. A manipulator according to claim 2,
wherein the turning action part, the passive part, the extension/contraction part and the distal-end action part are located in this order from the operating unit side.

4. A manipulator according to any one of claims 1 to 3, wherein
the operating unit includes a distance adjustment unit for adjusting a distance between a distal end of the insert part and a subject of interest, and
the control unit controls the driving unit in association with a distance set by the distance adjustment unit.

5. A manipulator according to any one of claims 1 to 4, further comprising
a distance measurement unit for sensing a distance between a distal end of the insert part and a subject of interest,
wherein the control unit controls the driving unit in association with information sensed by the distance measurement unit.

6. A manipulator according to any one of claims 1 to 5,
wherein the insert part is provided at a distal end on the turning action part side with a stopper having a diameter larger than that of the insert part.

7. A manipulator according to any one of claims 1 to 6,
wherein the operating unit includes a mode switchover instruction unit adapted to switch between a manual mode and a control mode in which the control unit controls the driving unit in association with operation of the operating unit.

8. A manipulator according to any one of claims 1 to 7,
wherein the control unit implements control such that the distal-end part turns to the subject of interest after control.

9. A manipulator system, **characterized by** comprising:
a manipulator according to any one of claims 1 to 8 and including an endoscope,
an image processor adapted to apply image processing to an image signal obtained from the endoscope,
and
a monitor adapted to display an image signal transmitted from the image processor.
